# EUROPEAN PATENT SPECIFICATION

(11) **EP 3 969 581 B1**
(45) Date of publication and mention of the grant of the patent: **08.10.2025**
(21) Application number: 20806525.0
(22) Date of filing: 13.05.2020
(51) Int. Cl.: C12N 15/00, C12N 15/09, C12N 15/10, C12N 15/11

(54) **CAPTURE AND ANALYSIS OF TARGET GENOMIC REGIONS**
ERFASSUNG UND ANALYSE VON GENOMISCHEN ZIELREGIONEN
CAPTURE ET ANALYSE DE RÉGIONS GÉNOMIQUES CIBLES

(30) Priority: 13.05.2019 US 201962846988 P
(43) Date of publication of application: 23.03.2022
(73) Proprietor: LGC Genomics, LLC, Alexandria, MN 56308-3339 (US)
(72) Inventor: NEVES, Leandro, Gomide, Gainesville, FL 32601 (US)
(74) Representative: Cabinet Becker et Associés
(86) International application number: PCT/US2020/032615
(87) International publication number: WO 2020/232081

(56) References cited:
- WO-A2-2015/188192
- US-A1- 2006 292 611
- US-A1- 2009 005 252
- US-A1- 2013 316 917
- US-A1- 2017 211 099
- COLLINS FRANCIS S ET AL: "Directional cloning of DNA fragments at a large distance from an initial probe: A circularization method (gene mapping/chromosome walking/suppressor tRNA) Contributed by", GENETICS, 1 November 1984 (1984-11-01), pages 6812 - 6816, XP093044214, Retrieved from the Internet <URL:https://www.ncbi.nlm.nih.gov/pmc/articles/PMC392022/pdf/pnas00622-0254.pdf> [retrieved on 20230504]

## Description

### BACKGROUND OF THE INVENTION

Capture and analysis, such as sequencing of target genomic regions is extremely important in diverse biotechnological fields ranging from agronomy, taxonomy to medicine. For example, sequencing target regions in a genome is important in precision and individualized medicine, where understanding the genetic diversity in specific regions of the genome can be linked to phenotypic information about a subject's health, which in turn can dictate treatment options. Also, sequencing target regions of a genome is important in agronomical applications for improving desirable traits in plants, such as seed and food production.

Several methods exist for the analyzing target genomic DNA or RNA sequences. For example, targeted amplification prior to sequencing is common and multiple methods are available to isolate and/or amplify target regions of the genome. As a common theme, the methods known in the art utilize variations of polymerase chain reaction (PCR), ligation-chain reaction or probe hybridization to isolate and amplify target genomic regions. Current methods for isothermal detection of genetic material can be performed, for example, using loop-mediated isothermal amplification (LAMP) or strand displacement amplification. However, these techniques are difficult to optimize for different targets and are not suitable for analyzing long targets. Also, these isothermal techniques are difficult or impossible to be multiplexed for a high number of targets and are not suitable for easily sequencing the amplified products. Characterizing, particularly, detecting and sequencing longer stretches of DNA sequences, for example, at least about 1-50 kilobase (kb), is of particular interest.

WO 2015/188192 A2 discloses a method of analysing DNA methylation using methylation sensitive restriction enzymes followed by circularisation of the cleavage fragments using splint oligonucleotides. However, such detection and sequencing are not readily achieved with the conventional methods. Therefore, improved methods for characterizing, such as detecting and sequencing target genomic regions, particularly, detecting and sequencing long stretches of target genomic regions, are desirable.

### BRIEF SUMMARY OF THE INVENTION

The present invention is as defined in the claims.

Certain embodiments of the invention provide materials and methods for capturing target genomic regions and optionally, further analyzing, such as by detecting and/or sequencing. The materials and methods disclosed herein are particularly suitable for analyzing target genomic regions containing at least about 1 kb, preferably, at least about 10 kb, even more preferably, at least about 30 kb, or most preferably, at least about 50 kb.

According to the methods disclosed herein, target genomic regions can be isolated based on cleavage at two specific recognition sites, each recognition site containing a minimum of between about 10 and about 30 nucleotides. The two recognition sites flank the target genomic region. The cleaved target genomic region can then be captured using an oligonucleotide, referenced herein as a "bridge oligo". A bridge oligo has sequences at the 3' and the 5' ends that hybridize to the sequences at the 3' and 5' ends, respectively, of the cleaved target genomic region. The captured target genomic regions can then be analyzed, for example, detected and/or sequenced, such as via amplification and sequencing.

Accordingly, certain embodiments of the invention provide methods for capturing a target genomic region from a genetic material, comprising:
a. cleaving the target genomic region from the genetic material using one or more endonucleases having a first recognition site and a second recognition site, each recognition site comprising a sequence of between 10 and 80 nucleotides, preferably of between 10 and 30 nucleotides, wherein the recognition sites flank the target genomic region,
b. denaturing the cleaved genetic material into single stranded form, and
c. capturing the target genomic region in the single stranded form by hybridizing the target genomic region to a bridge oligo, the bridge oligo comprising sequences at the 3' and the 5' that hybridize to the 3' and 5' ends, respectively, of the target genomic region in the single stranded form.

The captured target genomic region can be further analyzed, for example, detected or sequenced. Such analysis can comprise the steps of:
d. ligating the free ends of the single stranded target genomic region hybridized to the bridge oligo to produce a single stranded circular target genomic region that is hybridized to the bridge oligo,
e. optionally, degrading non-circularized genetic material,
f. optionally, amplifying the target genomic region by nucleic acid amplification to produce multiple copies of the target genomic region, and
g. analyzing the amplified target genomic region.

In preferred embodiments, the cleavage of the target genomic region at the recognition sites is performed using a first and a second programmable endonuclease, such as Clustered Regularly Interspaced Short Palindromic Repeats (CRISPR) associated protein 9 endonucleases (Cas9 endonuclease), for example, a first Cas9 endonuclease comprising a first guide RNA (gRNA) having a sequence complementary to the first recognition site and a second Cas9 endonuclease comprising a second gRNA having a sequence complementary to the second recognition site.

In further embodiments, the target genomic region hybridized to the bridge oligo is amplified via an amplification reaction, for example, an isothermal amplification reaction, preferably, via a polymerase chain reaction or rolling circle amplification (RCA) reaction. When only the bridge oligo is used as a primer for amplification, a single stranded amplification product is produced via RCA, which comprises concatenated copies of the target genomic region in single stranded form. One or more primers in addition to the bridge oligo can also be used in an RCA reaction to produce a double stranded amplification product, which comprises concatenated copies of the target genomic region in double stranded form. A conventional PCR reaction can also be used to amplify the target molecules using appropriate primers that bind to the target regions or to a common region introduced by the bridge oligo.

The amplified target genomic region can be detected using techniques known in the art, for example, using a labeled probe complementary to a sequence within the target genomic region. The amplified target genomic region can also be sequenced using techniques known in the art, for example, nanopore sequencing (Oxford Nanopore Technologies^{™}), reversible dye-terminator sequencing (Illumina^{™}) and Single Molecule Real-Time (SMRT) sequencing (PacBio^{™}).

The materials and methods disclosed herein can be modified to capture, and optionally, analyze, multiple target genomic regions, for example, in a multiplex reaction. In such embodiments, multiple pairs of target recognition sites are designed to cleave multiple target genomic regions and these multiple target genomic regions can be captured via a plurality of bridge oligos, each of the plurality of bridge oligos specifically designed to capture a target genomic region. In certain embodiments, the plurality of bridge oligos can be immobilized on a solid substrate, such as a chip. The multiple target genomic regions so captured can be detected or sequenced using techniques known in the art.

Further embodiments of the invention also provide kits for carrying out the methods of the invention. The kits of the invention comprise specific bridge oligos as defined in the claims, which are designed to capture the one or more target genomic regions, and optionally one or more endonucleases designed to cleave one or more target genomic regions. The kits of the invention also comprise one or more guide molecules as defined in the claims, preferably in the form of DNA or RNA, and which are designed to cleave one or more target genomic regions. Such kits can also comprise one or more Cas9 endonucleases.

The kits can further comprise polymerases, ligase, primers and other reagents for amplifying the captured one or more target genomic regions. Even further, the kits can provide instructions to perform the methods of the invention.

### BRIEF DESCRIPTION OF THE DRAWINGS

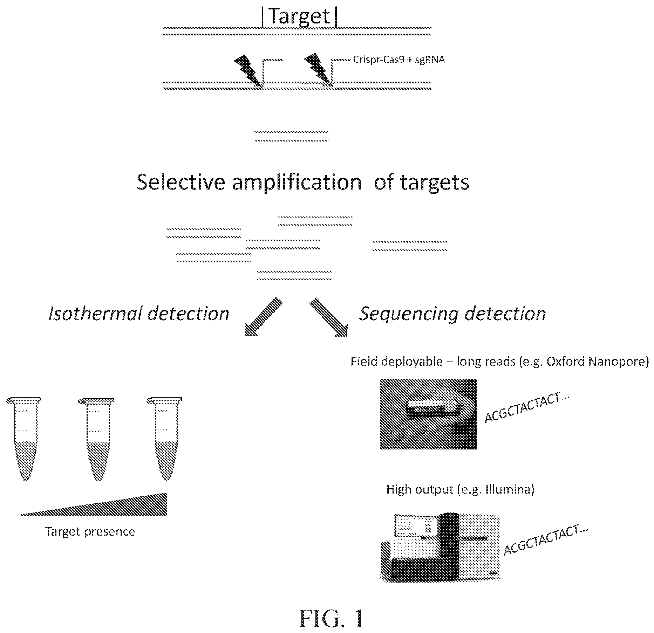

**FIG. 1****.** Overview of the methods to capture a target genomic region and two examples of analyzing the target genomic region, namely, detection or sequencing.

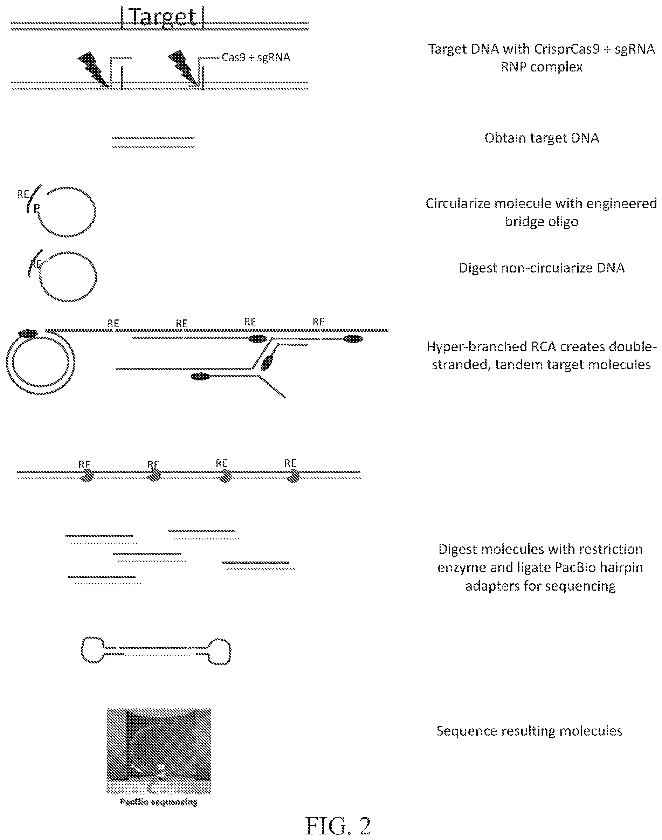

**FIG. 2****.** An example of sequencing a captured target genomic region using Single Molecule Real-Time (SMRT) sequencing (PacBio^{™}).

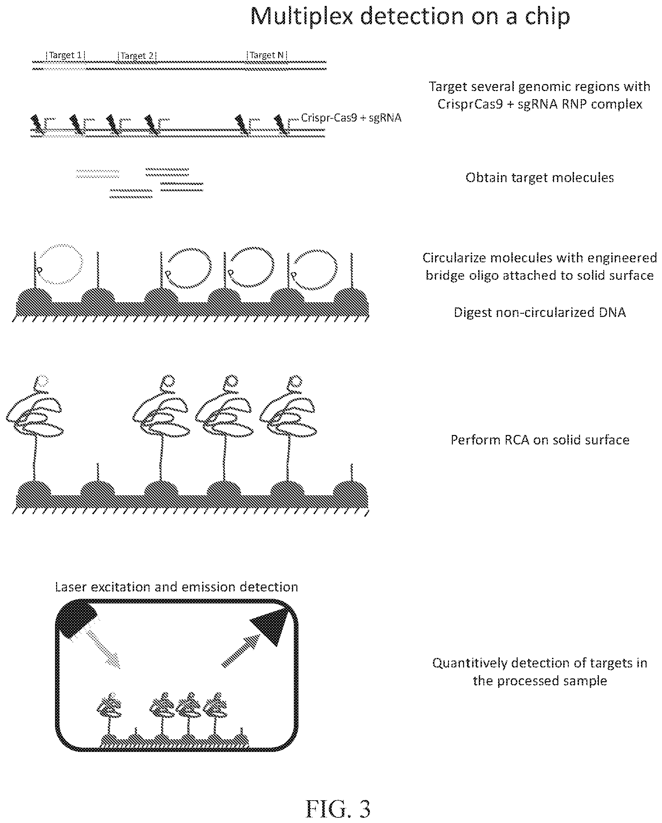

**FIG. 3****.** An example of the detection of multiple target genomic regions using an array of bridge oligos designed to capture multiple target genomic regions.
**FIG. 4****.** An example of amplification of circularized target molecule via PCR. Primers are illustrated containing adapters required for sequencing using reversible dye-terminator sequencing (IlluminaTM). A) Overview of the process. B) Structure of the bridge oligo needed to capture the sequences and provide landing sites for primers. C) Structure of the primers to allow for simultaneous amplification of the molecule, incorporation of unique sample identifier, and incorporation of the necessary structure for dye-terminator sequencing.
**FIG. 5****.** An example of designing recognition sites and bridge oligos based on two gRNAs that bind to the same strand of a double stranded genome.
**FIG. 6****.** An example of designing recognition sites and bridge oligos based on two gRNAs that bind to the opposite strands of a double stranded genome.
**FIG. 7****.** Another example of designing recognition sites and bridge oligos based on two gRNAs that bind to the opposite strands of a double stranded genome.
**FIG. 8****.** Another example of designing recognition sites and bridge oligos based on two gRNAs that bind to the same strand of a double stranded genome.
**FIG. 9****.** Examples of different approaches to ligate the ends of the target molecules to form a circular single-stranded structure.
**FIG. 10****.** An example of an RCA reaction resulting from a bridge oligo with a non-complementary 5' end section.
**FIG. 11****.** An example of utilizing a single programmable endonuclease and a primer extending from a target to form a circular single-stranded molecule and a bridge oligo.

### BRIEF DESCRIPTION OF THE SEQUENCES

SEQ ID NO: 1: Exemplary region to be studied according to the methods of the invention.
SEQ ID NO: 2: Exemplary first recognition site.
SEQ ID NO: 3: Exemplary second recognition site.
SEQ ID NO: 4: Exemplary target genomic region.
SEQ ID NO: 5: Sequence at the 5' end of the target genomic region of SEQ ID NO: 4.
SEQ ID NO: 6: Sequence at the 3' end of the target genomic region of SEQ ID NO: 4
SEQ ID NO: 7: Sequence at the 5' end of the bridge oligo designed to capture target genomic region of SEQ ID NO: 4.
SEQ ID NO: 8: Sequence at the 3' end of the bridge oligo designed to capture target genomic region of SEQ ID NO: 4.
SEQ ID NO: 9: A bridge oligo designed to capture the target genomic region of SEQ ID NO: 4.

### DETAILED DISCLOSURE OF THE INVENTION

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising". The transitional terms/phrases (and any grammatical variations thereof) "comprising", "comprises", "comprise", "consisting essentially of", "consists essentially of", "consisting" and "consists" can be used interchangeably.

The phrases "consisting essentially of" or "consists essentially of" indicate that the described embodiment encompasses embodiments containing the specified materials or steps and those that do not materially affect the basic and novel characteristic(s) of the described embodiment.

The term "about" means within an acceptable error range for the particular value as determined by one of ordinary skill in the art, which will depend in part on how the value is measured or determined, *i.e.,* the limitations of the measurement system. In the context of the lengths of polynucleotides where the terms "about" are used, these polynucleotides contain the stated number of bases or base-pairs with a variation of 0-10% around the value (X ± 10%).

In the present disclosure, ranges are stated in shorthand, so as to avoid having to set out at length and describe each and every value within the range. Any appropriate value within the range can be selected, where appropriate, as the upper value, lower value, or the terminus of the range. For example, a range of 0.1-1.0 represents the terminal values of 0.1 and 1.0, as well as the intermediate values of 0.2, 0.3, 0.4, 0.5, 0.6, 0.7, 0.8, 0.9, and all intermediate ranges encompassed within 0.1-1.0, such as 0.2-0.5, 0.2-0.8, 0.7-1.0, *etc.* Values having at least two significant digits within a range are envisioned, for example, a range of 5-10 indicates all the values between 5.0 and 10.0 as well as between 5.00 and 10.00 including the terminal values. When ranges are used herein, such as for the size of the polynucleotides, the combinations and sub-combinations of the ranges (*e.g*., subranges within the disclosed range) and specific embodiments therein, are explicitly included.

The term "organism" as used herein includes viruses, bacteria, fungi, plants and animals. Additional examples of organisms are known to a person of ordinary skill in the art and such embodiments are within the purview of the invention. The assays described herein can be useful in analyzing any genetic material obtained from any organism.

The term "genome", "genomic" or "genetic material" and other grammatical variations thereof as used herein refers to genetic material from any organism. A genetic material can be viral genomic DNA or RNA, nuclear genetic material, such as genomic DNA or genetic material present in cell organelles, such as mitochondrial DNA or chloroplast DNA. It can also represent the genetic material coming from a natural or artificial mixture or several organisms.

The phrase "long target genomic regions" used herein refers to a target genomic region having at least about 1 kb, preferably, at least about 10 kb, even more preferably, at least about 30 kb, or most preferably, at least about 50 kb.

The materials and methods disclosed herein for characterizing target genomic regions, particularly, long target genomic regions, solve the problems associated with conventional methods for capture and detection of target genomic regions, particularly, long target genomic regions.

The invention provides methods for capturing a target genomic region from a genetic material. The methods comprise the steps of:
a. cleaving the target genomic region from the genetic material using one or more endonucleases having a first recognition site and a second recognition site, each recognition site comprising a sequence of between 10 and 80 nucleotides, preferably of between 10 and 30 nucleotides, wherein the recognition sites flank the target genomic region,
b. denaturing the cleaved genetic material into single stranded form, and
c. capturing the target genomic region in the single stranded form by hybridizing the target genomic region to a bridge oligo, the bridge oligo comprising sequences at the 3' and 5' ends that hybridize to the 3' and 5' ends, respectively, of the target genomic region in the single stranded form.

The captured target genomic region can be further analyzed, for example, detected or sequenced. Such analysis can comprise the steps of:
d. ligating the free ends of the single stranded target genomic region hybridized to the bridge oligo to produce a single stranded circular target genomic region that is hybridized to the bridge oligo,
e. optionally, degrading non-circularized genetic material,
f. optionally, amplifying the target genomic region by nucleic acid amplification to produce multiple copies of the target genomic region, and
g. analyzing the amplified target genomic region.

As used herein, "a target genomic region" is a region of interest in the genome of an organism. Such region is flanked by a first recognition site and a second recognition site. Each of the first and the second recognition sites comprises a sequence of a minimum of between about 10 to about 30 nucleotides.

The first and the second recognition sites are selected based on the target genomic region. Typically, unique sequences that flank the target genomic region are selected as the first and the second recognition sites. Uniqueness of these sequences ensures that these sequences are less likely to occur elsewhere in the genome, thus minimizing non-specific cleavage and avoids capture of regions other than the target genomic region. Also, the minimum lengths of the first and the second recognition sites are preferably between about 10 and about 30 nucleotides, which ensure that these sequences occur rarely in a genome, thus also minimizing non-specific cleavage and capture of regions other than the target genomic region. The sequences of the first and the second recognition sites can be identical to each other or different from each other. A person of ordinary skill in the art can determine appropriate sequences for the first and the second recognition sites based on the sequence of the target genomic region and the available genomic sequence for a particular organism, for example, from a genome sequence database.

The cleavage of a genetic material at the recognition sites is performed using one or more endonucleases that cleave the phosphodiester bond (cut) in the genetic material at the specific recognition sites. The endonucleases can be restriction endonucleases. Certain restriction endonucleases that cut recognition sites comprising a minimum of between about 10 and about 30 nucleotides, for example, up to about 80 nucleotides, include meganucleases, such as homing endonucleases from the LAGLIDADG family, GIY-YIG family, HNH family, His-Cys box family and PD-(D/E)XK family. Additional examples of restriction endonucleases that cut recognition sites comprising a minimum of between about 10 and about 30 nucleotides, for example, up to about 80 nucleotides, are known in the art and such embodiments are within the purview of the invention.

In preferred embodiments, cleavage at the recognition sites is performed using one or more programmable endonucleases. The term programmable endonuclease is used to describe different classes of enzymes that can be targeted to cleave a specific region of a DNA or RNA molecule. Thus, a programmable endonuclease is an endonuclease that can be designed or programmed to cleave a nucleotide sequence of interest. For example, a programmable endonuclease can comprise of target recognition portion and endonuclease portion, where a common endonuclease portion can be combined with any target recognition portion to cleave a nucleotide sequence of interest.

In one embodiment, the programmable endonucleases are targeted by a guide RNA (gRNA), a guide DNA (gDNA) or by a structure formed between a guide molecule and the target (Varshney and Burgess, 2016). For example, Cas9 are programmable endonucleases, as they cleave double stranded genetic material by making a double stranded break at a specific location at a recognition site (Jinek et al., 2012). A gRNA having a specific sequence complementary to the sequence of the recognition site directs the Cas9 endonucleases to the recognition site. It has also been shown that gDNA or gRNA molecules can be used to target some types of argonaute proteins (Hegge et al., 2017; Swarts et al., 2015). Additional examples of programmable endonucleases include Cpf1, C2c1, C2c2, C2c, RNA- or DNA-guided Argonaute proteins, structure-guided endonucleases, among others.

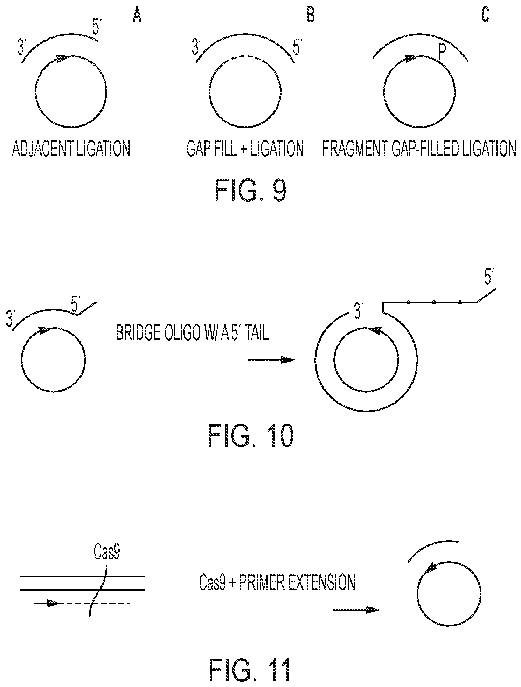

Novel proteins can also be engineered to cleave DNA based on recognizing specific DNA structures formed between a gDNA and the target sequence, such as a 3' end mismatch (Xu et al., 2016). Typically, in the methods of the invention, two Cas9 endonucleases are used to cleave a target genomic region from a genetic material, namely, a first Cas9 endonuclease that cuts DNA at a first recognition site based on a first gRNA having a sequence complementary to the first recognition site and a second Cas9 endonuclease that cuts DNA at a second recognition site based on a second gRNA having a sequence complementary to the second recognition site. The complex containing the gRNA and the components of the Cas9 endonuclease is called the ribonucleoprotein (RNP) complex. In some instances, only one RNP is required to cleave one strand of a double stranded DNA and the selection point on the other strand of the double stranded DNA is a result of a primerextension reaction (Figure 11).

The use of programmable endonucleases, such as Cas9, provides significant improvements over other methods known in the art to produce, capture or manipulate circular molecules from target regions (Dahl et al., 2007; Fredriksson et al., 2007). First, the use of common restriction enzymes limits the target genomic regions that can be analyzed because a combination of restriction enzymes might not exist that cut near the target region without cutting within the target region. This is particularly problematic as the number of sites increases. Furthermore, even if such combinations of restriction enzymes exist, they are likely to change every time a new target is envisioned, making the process very difficult to scale. Conversely, using programmable endonucleases allows for virtually any region to be targeted in a scalable process, because the design of unique guide oligos and their synthesis is well-known in the art. Second, the use of PCR instead of endonucleases to extract the sites to be circularized, as done in Fredriksson et al., (2007), limits the size of the target molecules because PCR tends to work best for fragments under 1 Kb. In addition, PCR requires laborious optimization and can be very difficult, cost-prohibitive or impossible to be carried out for a large number of target regions in parallel.

The sequences of the first and the second recognition sites are expressed in this disclosure from 5' to 3' direction. Therefore, a sequence toward the 5' end of the gRNA is complementary to the sequence of the recognition site. In addition to a sequence complementary to a recognition site, a Cas9-gRNA RNP complex also recognizes a short conserved sequence motif of about two to five nucleotides, typically, three nucleotides, located adjacent on the non-complementary strand of the target DNA and to the 3' end of the sequence of the gRNA that is complementary to the recognition site, which is called protospacer adjacent motif (PAM). PAM is critical in the gRNA binding to the recognition site as well as Cas9 mediated cleavage of the target genetic material, although engineered Cas9 enzymes are envisioned that lack this requirement of a PAM site.

One example of a PAM is the sequence NGG, but different Cas9 enzymes are known to require different PAM sites and enzymes are being modified to tolerate variable PAM sites (Hu et al., 2018). Therefore, in certain embodiments of gRNA useful in the materials and methods disclosed herein, a recognition site is designed so that the sequence of the recognition site on the genetic material is immediately followed toward the 3' side of the non-complementary strand by the sequence 5'-NGG-3'.

When the gRNA binds to the recognition site, the Cas9 endonuclease creates a double stranded break in the double stranded genetic material at three nucleotides toward the 5' side of the NGG sequence on the non-complementary strand, i.e., starting from the 5' end and going towards the 3' end of the recognition site, Cas9 endonuclease makes a double stranded break between the third and the fourth nucleotide.

Once a genetic material is cleaved using appropriate endonucleases, the resultant mixture of the target genomic regions and the cleaved fragments of the genetic material are denatured to convert it into single stranded form, for example, by subjecting it to denaturation conditions. Typically, subjecting cleaved genetic material to denaturation conditions comprises subjecting it to an appropriate temperature in the presence of appropriate compounds, such as salt, dimethyl sulfoxide, sodium hydroxide, etc. and at appropriate pH. DNA can also be denatured using chemical treatment with NaOH or high salt concentration. In preferred embodiments, the cleaved genetic material is denatured by subjecting it to a temperature of: between 75°C to 115°C, preferably, between 80°C to 110°C, more preferably, between 85°C and 105°C and even more preferably, between 90°C and 100°C, and most preferably, about 95°C. A person of ordinary skill in the art can determine appropriate denaturation conditions and such embodiments are within the purview of the invention.

To capture the target genomic region separated from the genetic material, a specifically designed bridge oligo is contacted with the cleaved and denatured genetic material. Typically, the bridge oligo is a single stranded oligonucleotide. The bridge oligo comprises sequences at the 3' and 5' ends that hybridize to the sequences at the 3' and the 5' ends, respectively, of the single stranded target genomic region. A bridge oligo can also be a double stranded oligonucleotide having 3' and 5' end overhangs that hybridize to the sequences at the 3' and the 5' ends, respectively, of the single stranded target genomic region, which can be used to capture both strands of the target DNA molecule. The bridge oligos can be protected against nuclease degradation by the different ways known in the art so that they remain present in the reaction after a treatment with given nucleases, such as adding one or more phosphorothioate bond in its 3' and/or 5' ends, and adding inverted dT and inverted ddT at its 3' and 5' end, respectively.

The bridge oligo serves several purposes. The bridge oligo circularizes the correct target genomic regions generated by on-target cuts with the endonucleases. Because off-target cutting is expected in a reaction with endonucleases, a bridge oligo provides additional specificity by only allowing the correct molecules to be circularized. Also, the bridge oligo itself can serve as the primer for the subsequent amplification.

Further, the bridge oligo can be engineered to provide additional functionality, such as preparing the resulting molecules for sequencing. For example, a bridge oligo can be immobilized on a solid surface for detection on a chip or it can be biotinylated for recovery with streptavidin-bound beads. The 5' and 3' end of the bridge oligos can also have additional "tail" sequences that are non-complementary to the target region, creating common sequences that can be used to link to the molecule additional functionalities, such as providing sites for binding of primers for PCR, add biotin molecules or other modifications known in the art (Figure 10).

Between the sequences at the two ends that hybridize with the target genomic regions, a bridge oligo can further contain sequences, such as a restriction site specific for a rare-cutter restriction endonuclease, a primer binding sequence or a target for a programmable endonuclease. The rare-cutter restriction site or the target for a programmable endonuclease can be used to cleave individual target genomic regions from the concatenated copies of the target genomic region produced after nucleic acid amplification. Non-limiting examples of rare-cutter restriction endonucleases are described in PCT Publication WO 2009/079488, particularly, Table 1.

As used herein, "a rare-cutter restriction endonuclease" is an endonuclease whose restriction site occurs rarely in a genetic material. For example, for human genome, a rare-cutter restriction endonuclease is an endonuclease whose restriction site occurs on average every 50-100 kb, preferably, every 100-200 kb, or more preferably, every 200-400 kb, or even more preferably, every 400-600 Kb. Examples of rare-cutter restriction endonucleases for human genome and their restriction sites are given in Table 1 below:

**Table 1. Examples of human rare-cutter endonucleases and their restriction sites.**

| Restriction Enzyme | Recognition site | Frequency in Human genome (kb) |
|---|---|---|
| Not I | GCGGCCGC | 1000 |
| Xma III | CGGCCG | 100 |
| Sst II | CCGCGG | 100 |
| Sal I | GTCGAC | 100 |
| Nru I | TCGCGA | 300 |
| Nhe I | GCTAGC | 100 |

Additional rare-cutter endonucleases are described in, e.g., Restriction Endonucleases ((Nucleic Acids and Molecular Biology) by Pingoud (Editor), Springer; 1 ed. (2004)). Many rare-cutter endonucleases are also commercially available, such as roaming class of endonucleases, e.g., from New England BioLabs (Beverly, MA). Even further examples of rare-cutter endonucleases are known in the art and such embodiments are within the purview of the invention.

A primer binding sequence in a bridge oligo facilitates using an additional primer in a RCA reaction to amplify the target genomic regions and produce concatenated copies of the target genomic region in double stranded form, through hyper-branching of the original molecule.

The first and the second recognition sites can be present on the same strand of the genetic material or on the opposite strands of the genetic material.

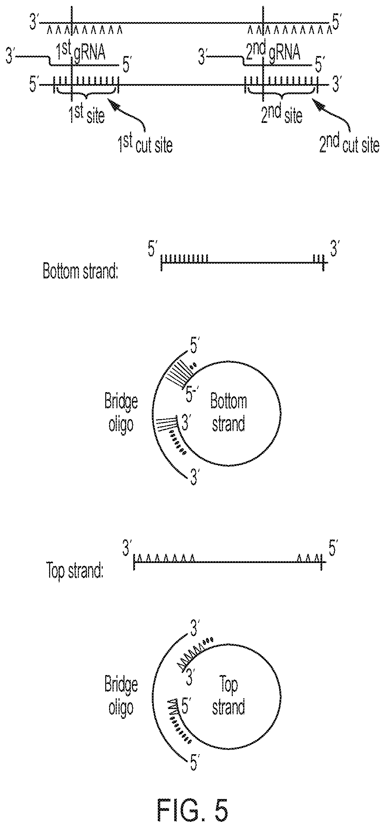

Figure 5 provides an example of designing recognition sites and bridge oligos based on two gRNAs that bind to the same strand of a double stranded genome. As shown in Figure 5, the bottom strand of the double stranded genomic DNA is selected for designing the recognition sites. The first gRNA binds to the first recognition site and the Cas9 endonuclease cuts the genomic DNA three nucleotides downstream of the 5' end of the first recognition site. The second gRNA binds to the second recognition site and the Cas9 endonuclease cuts the genomic DNA three nucleotides downstream of the 5' end of the second recognition site. Thus, the target genomic region is cleaved from the genomic DNA in double stranded form. This target genomic region has, at the end towards the first recognition site, all but the first three nucleotides from the first recognition site and has, at the end towards the second recognition site, the first three nucleotides from the second recognition site. This double stranded target genomic region can be converted to single stranded form and one or more bridge oligos can be designed to capture either or both of the strands.

To capture the bottom strand from Figure 5, a bridge oligo is designed to have, towards the 3' end, a sequence complementary to the first three nucleotides of the second recognition site and additional nucleotides that are complementary to the sequence present on the bottom strand beyond and adjacent to the 5' end of the second recognition site. This bridge oligo has, towards the 5' end, a sequence complementary to the first recognition site except the first three nucleotides and optionally, additional nucleotides that are complementary to the sequence present on the bottom strand beyond and adjacent to the 3' end of the first recognition site.

To capture the top strand from Figure 5, a bridge oligo is designed to have, towards the 3' end, the sequence of the first recognition site except the first three nucleotides and optionally, a sequence present on the bottom strand beyond and adjacent to the 3' end of the first recognition site. This bridge oligo has, towards the 5' end, the sequence of the first three nucleotides of the second recognition site and additional nucleotides that are present on the bottom strand beyond and adjacent to the 5' end of the second recognition site.

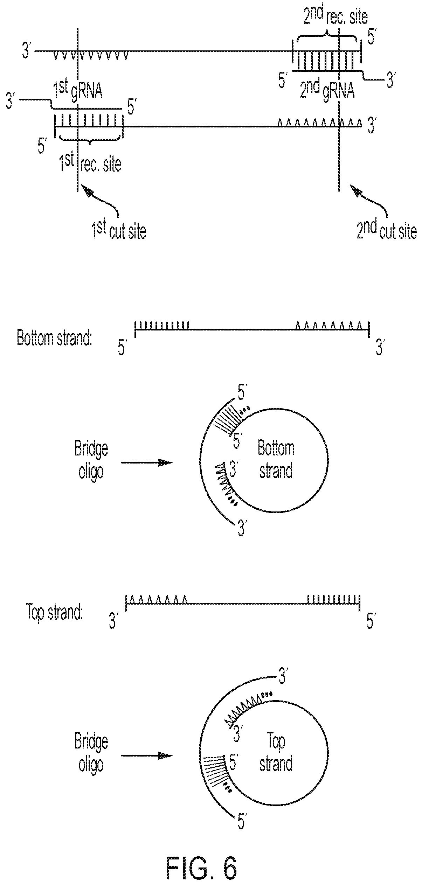

Figure 6 provides an example of designing recognition sites and bridge oligos based on two gRNAs that bind to the opposite strands of a double stranded genome. As shown in Figure 6, the bottom strand of the double stranded genomic DNA is selected for designing the first recognition site and the top strand of the double stranded genomic DNA is selected for designing the second recognition site. The first gRNA binds to the first recognition site on the bottom strand and the Cas9 endonuclease cuts the genomic DNA three nucleotides downstream of the 5' end of the first recognition site. Similarly, the second gRNA binds to the second recognition site on the top strand and the Cas9 endonuclease cuts the genomic DNA three nucleotides downstream of the 5' end of the second recognition site. Thus, the target genomic region is cleaved from the genomic DNA in double stranded form. This target genomic region has, at the end towards the first recognition site, the sequence of the first recognition site except the first three nucleotides, and has, at the end towards the second recognition site, the sequence of the second recognition site except the first three nucleotides. This double stranded target genomic region can be converted to single stranded form and one or more bridge oligos can be designed to capture either or both of the strands.

To capture the bottom strand from Figure 6, a bridge oligo is designed to have, towards the 3' end, the sequence of the second recognition site except the first three nucleotides and optionally, a sequence present on the top strand beyond and adjacent to the 3' end of the second recognition site. This bridge oligo has, towards the 5' end, the sequence complementary to the first recognition site except the first three nucleotides and optionally, a sequence complementary to the sequence present on the bottom strand beyond and adjacent to the 3' end of the first recognition site.

To capture the top strand from Figure 6, a bridge oligo is designed to have, towards the 3' end, the sequence of the first recognition site except the first three nucleotides and optionally, a sequence present on the bottom strand beyond and adjacent to the 3' end of the first recognition site. This bridge oligo has, towards the 5' end, a sequence complementary to the second recognition site except the first three nucleotides and optionally, a sequence complementary to the sequence present on the top strand beyond and adjacent to the 3' end of the second recognition site.

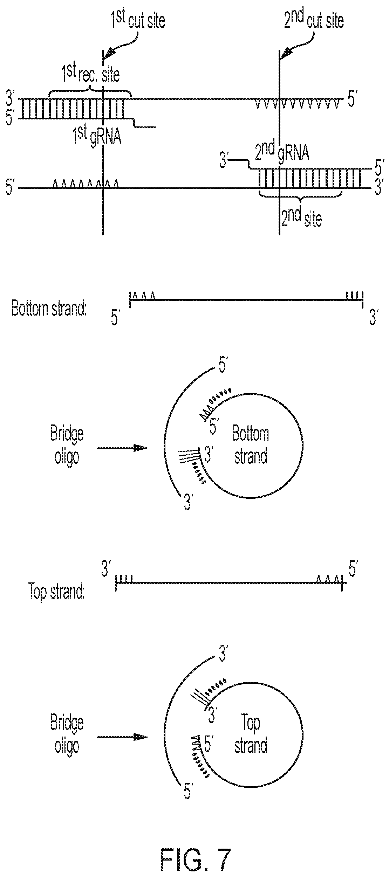

Figure 7 provides another example of designing recognition sites and bridge oligos based on two gRNAs that bind to the opposite strands of a double stranded genome. As shown in Figure 7, the top strand of the double stranded genomic DNA is selected for designing the first recognition site and the bottom strand of the double stranded genomic DNA is selected for designing the second recognition site. The first gRNA binds to the first recognition site on the top strand and the Cas9 endonuclease cuts the genomic DNA three nucleotides downstream of the 5' end of the first recognition site. Similarly, the second gRNA binds to the second recognition site on the bottom strand and the Cas9 endonuclease cuts the genomic DNA three nucleotides downstream of the 5' end of the second recognition site. Thus, the target genomic region is cleaved from the genomic DNA in double stranded form. This target genomic region has, at the end towards the first recognition site, the first three nucleotides of the first recognition site and has, at the end towards the second recognition site, the first three nucleotides of the second recognition site. This double stranded target genomic region can be converted to single stranded form and one or more bridge oligos can be designed to capture either or both of the strands.

To capture the bottom strand from Figure 7, a bridge oligo is designed to have, towards the 3' end, a sequence complementary to the first three nucleotides of the second recognition site and additional nucleotides that are complementary to the sequence present on the bottom strand beyond and adjacent to the 5' end of the second recognition site. This bridge oligo has, towards the 5' end, the sequence of the first three nucleotides of the first recognition site and additional nucleotides that are present on the top strand beyond and adjacent to the 5' end of the first recognition site.

To capture the top strand from Figure 7, a bridge oligo is designed to have, towards the 3' end, a sequence complementary to the first three nucleotides of the first recognition site and additional nucleotides that are complementary to the sequence present on the top strand beyond and adjacent to the 5' end of the first recognition site. This bridge oligo has, towards the 5' end, the sequence of the first three nucleotides of the second recognition site and additional nucleotides that are present on the bottom strand beyond and adjacent to the 5' end of the second recognition site.

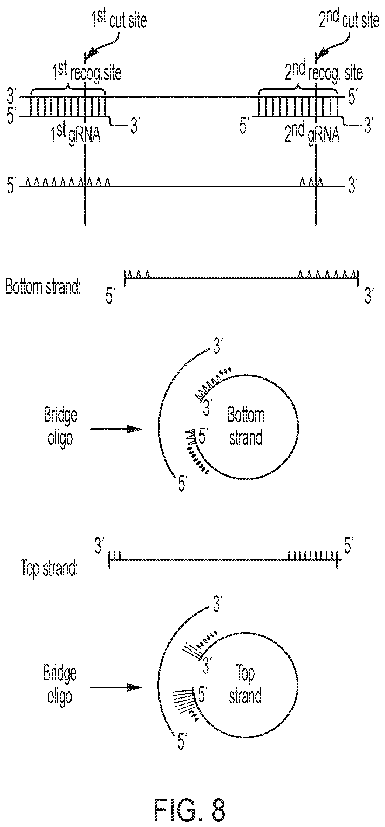

Figure 8 provides another example of designing recognition sites and bridge oligos based on two gRNAs that bind to the same strand of a double stranded genome. As shown in Figure 8, the top strand of the double stranded genomic DNA is selected for designing the first recognition site and the second recognition site. The first gRNA binds to the first recognition site on the top strand and the Cas9 endonuclease cuts the genomic DNA three nucleotides downstream of the 5' end of the first recognition site. Similarly, the second gRNA binds to the second recognition site on the top strand and the Cas9 endonuclease cuts the genomic DNA three nucleotides downstream of the 5' end of the second recognition site. Thus, the target genomic region is cleaved from the genomic DNA in double stranded form. This target genomic region has, at the end towards the first recognition site, the first three nucleotides at the 5' end of the first recognition site and has, at the end towards the second recognition site, all but the first three nucleotides at the 5' end of the second recognition site. This double stranded target genomic region can be converted to single stranded form and one or more bridge oligos can be designed to capture either or both of the strands.

To capture the bottom strand from Figure 8, a bridge oligo is designed to have, towards the 3' end, the sequence of the second recognition site except for the first three nucleotides and optionally, the sequence present on the top strand beyond and adjacent to the 3' end of the second recognition site. This bridge oligo has, towards the 5' end, the sequence of the first three nucleotides of the first recognition site and additional nucleotides that are present on the top strand beyond and adjacent to the 5' end of the first recognition site.

To capture the top strand from Figure 8, a bridge oligo is designed to have, towards the 3' end, a sequence complementary to the first three nucleotides of the first recognition site and additional nucleotides that are complementary to the sequence present on the top strand beyond and adjacent to the 5' end of the first recognition site. This bridge oligo has, towards the 5' end, a sequence complementary to the second recognition site except the first three nucleotides and optionally, a sequence complementary to the sequence present on the top strand beyond and adjacent to the 3' end of the second recognition site.

The sequences of the bridge oligos represented in Figures 5-8 and described in the preceding paragraphs do not need to be perfectly complementary or identical to the corresponding sequences as long as the bridge oligo can hybridize with the corresponding sequences on the target genomic regions. Therefore, certain degree of mismatch can be allowed and such variation is within the purview of the invention.

The term "hybridizes with" indicates that the two sequences are sufficiently complementary to each other to allow hybridization between the two sequences. Sequences that hybridize with teach other can be perfectly complementary but can also have mismatches to a certain extent. Therefore, the sequences at the 5' and 3' ends of a bridge oligo may have a few mismatches with the corresponding sequence at the 5' and 3' ends of the target genomic region as long as the bridge oligo can hybridize with and capture the target genomic region. Depending upon the stringency of hybridization, a mismatch of about 5% to about 20% between the two complementary sequences would allow for hybridization between the two sequences. Typically, high stringency conditions have higher temperature and lower salt concentration and low stringency conditions have lower temperature and higher salt concentration. High stringency conditions for hybridization are preferred, and therefore, the sequences at the 3' and 5' ends of a bridge oligo are preferred to be perfectly complementary to the sequences at the 3' and 5' ends, respectively, of the target genomic region.

The captured target genomic region can be further analyzed, for example, detected or sequenced. Such analysis can comprise the steps of:
d. ligating the free ends of the single stranded target genomic region hybridized to the bridge oligo to produce a single stranded circular target genomic region that is hybridized to the bridge oligo,
e. optionally, degrading non-circularized genetic material,
f. optionally, amplifying the target genomic region by nucleic acid amplification to produce multiple copies of the target genomic region, and
g. analyzing the amplified target genomic region.

In some embodiments, ligating the free ends of the single stranded target genomic region hybridized to the bridge oligo to produce a single stranded circular target genomic region that is hybridized to the bridge oligo is performed by using a ligase (Figure 9A). When additional sequences are present in the bridge oligo beyond the sequences complementary to ends of the target genomic region, ligating the free ends of the single stranded target genomic region also comprises nucleic acid synthesis to fill the gap between the two ends of the target genomic region using an appropriate DNA polymerase enzyme (Figure 9B). The gap between the ends of the target can also be filled by hybridizing a single-stranded oligo to the bridge oligo in the gap and ligating the resulting molecules together (Figure 9C). Ligase enzymes suitable for ligating the free ends of the single stranded target genomic are known in the art and include T4 DNA ligase, Ampligase, T7 DNA ligase and Taq DNA ligase.

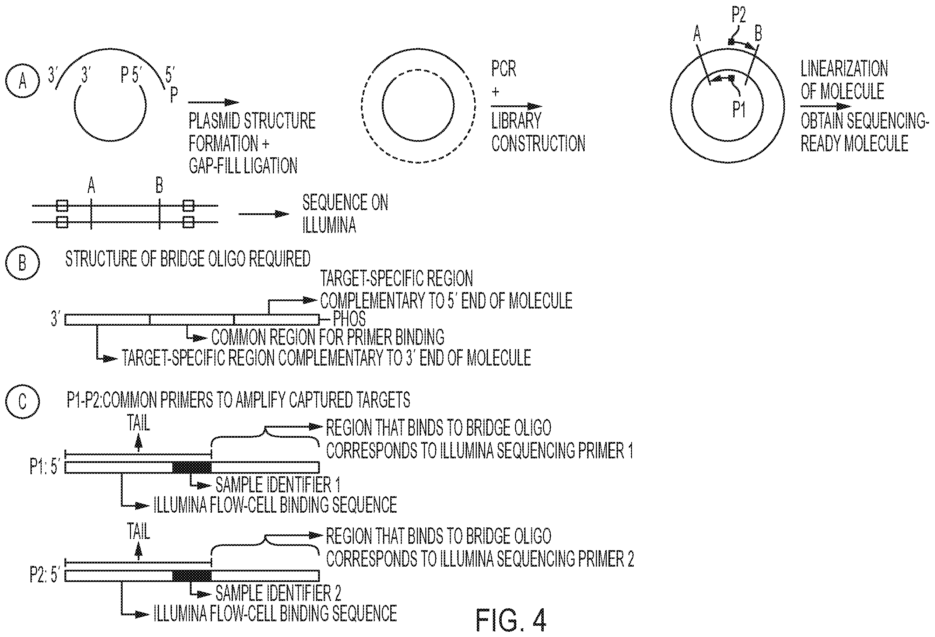

In certain embodiments, the properly circularized target sequences can be converted into a double-stranded vector. This can be done by supplementing the reaction with a DNA polymerase without strand-displacement capabilities and DNA ligase, which will fill synthesize a second strand complementary to the circularized target (Figure 4).

In certain embodiments, once the target genomic regions are properly circularized, the remaining genetic material, for example, uncleaved genomic DNA and off-targets genomic fragments, are removed, for example, degraded. This can be done by a combination of exonucleases that degrade the nucleic acids from their exposed 3' or 5' terminus, whether single-stranded or double-stranded, DNA or RNA. Thus, a treatment with appropriate exonucleases leaves only the circularized target molecules and greatly reduces the difficulty of the subsequent steps.

In certain embodiments, the properly circularized target sequences, separated from the rest of the genetic material via a treatment with an exonuclease and hybridized to the bridge oligo, can be amplified via nucleic acid amplification. In certain preferred embodiments, the amplification can be done via PCR, using the sequences in the bridge oligo to design primers that can amplify the circular molecules and convert them back into linear molecules. This is particularly suitable for short stretches for DNA (<10 Kb), where PCR is most efficient. The bridge oligos for multiple targets can contain a common region non-complementary to the target that is used to drive the PCR reaction with a pair of common primers for all fragments in parallel (Figure 4). In other preferred embodiments, such amplification is an isothermal amplification, for example, rolling circle amplification (RCA). The isothermal amplification facilitates amplification of long stretches of DNA, for example, multiple copies of target genomic regions, each copy containing at least about 10-50 kb can be produced.

In a conventional RCA reaction, padlock synthetic probes are used to hybridize a target nucleic acid sequence and create a circle (Nilsson et al., 1994). The circle is then amplified in an RCA reaction. In the instant invention, a circularized target sequence captured using a bridge oligo is amplified via an RCA reaction. In such RCA reaction, the bridge oligo acts as the primer for the RCA reaction, which linearly amplifies the target sequence creating long, single-stranded, tandem repeats of the target sequence. The amplified single stranded target sequences can then be used for subsequent analysis. The amplified target sequences can also be converted into double-stranded form, for example by capturing both target strands and allowing their RCA products to reanneal.

In some embodiments, exponential amplification of the target circularized molecules can be obtained through hyper-branched RCA using one or more additional primers (beyond the bridge oligo itself) to generate double-stranded tandem repeats of the target sequence. The primers beyond the bridge oligo can be designed based on the sequences of the bridge oligo, based on the sequences present in the target genomic region, or be composed of a combination of random bases to amplify multiple positions of the circle.

Additional approaches to convert the single stranded captured target genomic regions into double-stranded form are known in the art and such embodiments are within the purview of the instant invention.

The target genomic regions of different sizes can be created and amplified by RCA, thereby bypassing limitations of conventional methods that depend on amplifying nucleic acids by PCR and, hence, are not effective for amplifying long target genomic regions. An important advantage provided by the methods disclosed herein is the detection and analysis of long target genomic regions, for example, genomic regions containing at least about 1 kb, preferably, at least about 15 kb, even more preferably, at least about 30 kb, or most preferably, at least about 50 kb.

In certain embodiments, the product of the RCA reaction can be analyzed to detect or sequence the target genomic region. For example, amplification product can be detected based on the turbidity of the reaction, fluorescence detection or labeled molecular beacons.

The term "label" refers to a molecule detectable by spectroscopic, photochemical, biochemical, immunochemical, chemical, or other physical means. For example, useful labels include fluorescent dyes (fluorophores), fluorescent quenchers, luminescent agents, electron-dense reagents, biotin, digoxigenin, ³²P and other isotopes or other molecules that can be made detectable, e.g., by incorporating into an oligonucleotide. The term includes combinations of labeling agents, e.g., a combination of fluorophores each providing a unique detectable signature, e.g., at a particular wavelength or combination of wavelengths.

Exemplary fluorophores include, but are not limited to, Alexa dyes (e.g., Alexa 350, Alexa 430, Alexa 488, etc.), AMCA, BODIPY 630/650, BODIPY 650/665, BODIPY-FL, BODIPY-R6G, BODIPY-TMR, BODIPY-TRX, Cascade Blue, Cy2, Cy3, Cy5, Cy5.5, Cy7, Cy7.5, Dylight dyes (Dylight405, Dylight488, Dylight549, Dylight550, Dylight 649, Dylight680, Dylight750, Dylight800), 6-FAM, fluorescein, FITC, HEX, 6-JOE, Oregon Green 488, Oregon Green 500, Oregon Green 514, Pacific Blue, REG, Rhodamine Green, Rhodamine Red, ROX, R-Phycoerythrin (R-PE), Starbright Blue Dyes (e.g., Starbright Blue 520, Starbright Blue 700), TAMRA, TET, Tetramethylrhodamine, Texas Red, and TRITC.

Certain embodiments of the invention provide detection in parallel of more than four different targets. For example, different bridge oligos can be immobilized onto a substrate, for example, a chip, where the coordinates of each bridge oligo and, therefore, of each target genomic region is known. Using such substrate, dozens, hundreds or even thousands of target sequences can be detected and analyzed in a multiplex reaction.

In additional embodiments, the product of the PCR or RCA reaction is sequenced. Various methods of sequencing can be used for sequencing the product of the PCR and RCA, such as using portable Nanopore Minion^{™} or benchtop machines, Nanopore Promethion^{™}, PacBio Sequel^{™} or Illumina HiSeq^{™}. The sequencing step can also be used for multiplex detection of several targets and/or polymorphism detection.

In certain embodiments, short properly circularized target sequences can be prepared for sequencing after PCR amplification (Figure 4). The PCR primers can be specific to the target regions or, preferably, be universal to all targets by designing them to amplify a common region introduced by the bridge oligo (Figures 4B-4C). Once amplified, adapter molecules can be added to the molecules to make them compatible with the sequencer being used, following the specific manufacture's recommendations. Alternatively, primers with "tails" non-complementary to the bridge oligo can be used to incorporate the necessary adapters during the PCR, making the process more efficient (Figures 4A and 4C). These primers may also contain a short unique sequence (4-16 nucleotides) that is added during PCR to link the sequencing data to the respective sample, commonly known as index or barcode (Figure 4C).

In certain embodiments, concatenated copies can be cleaved to produce individual copies of the target genomic region, for example, using a rare-cutter restriction enzyme to cut the concatenated copies at the restriction site introduced via the bridge oligo. Such cleavage produces multiple copies of the target genomic region, each having sticky ends.

The sticky ends can be used to conjugate the target genomic regions to an adapter sequence. For example, an adapter comprising overhangs complementary to the restriction site specific for the rare-cutter restriction enzyme can be mixed with the copies of the target genomic regions to produce a double stranded DNA comprising the target genomic region flanked by the adapters.

The term "adapter" as used herein refers to a known nucleotide sequence of between four to one hundred nucleotides, preferably, between ten to twenty nucleotides, and even more preferably, about fifteen nucleotides, depending on the sequencing technology being used. The adapter sequences once incorporated at the ends of the amplified copies of the target genomic regions can facilitate sequencing of the target genomic regions, for example, by providing binding sites for primers. In one embodiment, target genomic regions flanked by the adapters are sequenced using paired-end sequencing.

The term "paired-end sequencing" used herein refers to the sequencing technology where both ends of a fragment are sequenced using specific primer binding sites present on each of the ends of the double stranded polynucleotides. Paired-end sequencing generates high-quality sequencing data which is aligned using a computer software program to generate the sequence of the polynucleotide flanked by the two primer binding sites. Sequencing from both ends of a double stranded molecule allows high quality data from both ends of the double stranded molecule because sequencing from only one end of the molecule may cause the sequencing quality to deteriorate as longer sequencing reads are performed.

In the paired-end sequencing, the double stranded polynucleotides produced at the end of the adapter incorporation are sequenced using specific primers that bind to the two ends of the double stranded target genomic regions flanked by the adapters. A general description and the principle of paired-end sequencing is provided in Illumina Sequencing Technology, Illumina, Publication No. 770-2007-002.

Non-limiting examples of the paired-end sequencing technology are provided by Illumina MiSeq^{™}, Illumina MiSeqDx^{™} and Illumina MiSeqFGx^{™}. Additional examples of the paired-end sequencing technology that can be used in the assays of the invention are known in the art and such embodiments are within the purview of the invention.

In certain embodiments, the sticky ends of the cleaved copies of target genomic regions can be used to conjugate the target genomic regions with hairpin adapters. For example, a hairpin adapter comprising overhangs complementary to the restriction site specific for the rare-cutter restriction enzyme can be mixed with the copies of the target genomic regions to produce a double stranded DNA comprising the target genomic region flanked by the hairpin adapters.

As used herein, the phrase "hairpin adapter" refers to a polynucleotide containing a double stranded stem and a single stranded hairpin loop. The single stranded hairpin loop region of a hairpin adapter can provide primer binding site for sequencing. Thus, once a hairpin adapter hybridizes with both sticky ends of a target genomic sequence, it produces a double-stranded DNA template containing the target genomic region in the double stranded region capped by hairpin loops at both ends. Such template can be used for sequencing the target genomic region via Single Molecule Real-Time (SMRT) sequencing (PacBio^{™}).

Description and the principle of SMRT sequencing is provided in Pacific Biosciences (2018), Publication No.: BR108-100318.

In further embodiments, nanopore technology is used to sequence the target genomic regions. In certain such embodiments, the copies of target genomic regions are processed to sequence the target genomic regions as described, for example, in Nanopore Technology Brochure, Oxford Nanopore Technologies (2019), and Nanopore Product Brochure, Oxford Nanopore Technologies (2018).

In certain embodiments, multiple target genomic regions are captured and optionally, further analyzed, such as detected or sequenced. In such embodiments, a pair of gRNAs is designed for each target genomic region. For designing a plurality of gRNAs for a plurality of target genomic regions, the sequences of the gRNAs are selected so that the Cas9 endonuclease for one target genomic region does not disrupt other target genomic regions. Based on the known genomic sequences of the concerned organism, a person of ordinary skill in the art can determine appropriate combinations of recognition sites to specifically cleave and isolate multiple target genomic regions. The gRNAs can also be designed and synthesized with degenerate or wobble bases to allow for hybridization to multiple or uncertain locations. For example, this can be done to compensate for known polymorphisms in the gRNA target site, to add multiple degenerate bases to randomly hybridize to multiple positions of the genome, as well as to design gRNAs that hybridize to an unknown gene that encodes a known amino acid sequence based on the degeneracy of the genetic code. In such cases, the bridge oligo may also be adapted to contain the degenerate bases, if needed.

Accordingly, certain embodiments of the invention provide a method for capturing a plurality of target genomic regions from a genetic material. The methods comprise the steps of:
a. cleaving a plurality of target genomic regions from the genetic material using a plurality of pairs of endonucleases, each pair of endonucleases having a first recognition site and a second recognition site, each recognition site comprising a sequence of a minimum of between about 17 and about 24 nucleotides, wherein each pair of recognition sites flanks a target genomic region from the plurality of target genomic regions,
b. denaturing the cleaved genetic material into single stranded form, and
c. capturing the plurality of target genomic regions in the single stranded form by hybridizing the target genomic regions to a plurality of bridge oligos, wherein each bridge oligo comprises sequences at the 3' and 5' ends that hybridize to the 3' and 5' ends, respectively, of a target genomic region from the plurality of target genomic regions in single stranded form.

The aspects described above of capturing a target genomic region, for example, designing the recognition sites, endonucleases used in cleaving the genetic material and designing the bridge oligos for capturing the cleaved target genomic regions are also applicable to the instant methods of capturing a plurality of target genomic regions.

**In** one embodiment of capturing a plurality of target genomic regions, a substrate is provided having a plurality of bridge oligos conjugated to the substrate at specific known locations. A genetic material cleaved with a plurality of pairs of endonucleases is converted into the single stranded form and the plurality of target genomic regions in single stranded form is contacted with the substrate under appropriate conditions to allow hybridization between the bridge oligos conjugated to the substrate the plurality of target genomic regions. Once target genomic regions are hybridized to the corresponding bridge oligos and circularized, the target genomic regions present at specific locations can be further analyzed. **In** some embodiments, the capture target genomic regions are amplified using RCA and further detected, for example, using laser excitation and emission detection methods.

**In** certain embodiments, the plurality of target genomic regions are further analyzed, for example, detected or sequenced. Such analysis can comprise the steps of:
d. ligating the free ends of the single stranded target genomic regions hybridized to the plurality of bridge oligos to produce a plurality of single stranded circular genetic materials, each containing a target genomic region from the plurality of target genomic regions, hybridized to the corresponding bridge oligo,
e. optionally, removing non-circularized genetic material from the substrate,
f. optionally, amplifying the plurality of target genomic regions by nucleic acid amplification to produce multiple copies of each target genomic region, and
g. analyzing the amplified plurality of target genomic regions.

The aspects described above of analyzing a target genomic region, for example, ligating the free ends of the single stranded target genomic regions, detecting the target genomic regions or sequencing the target genomic regions are also applicable to the instant methods of analyzing a plurality of target genomic regions.

In certain embodiments, the non-circularized genetic material can be removed by degrading with exonucleases or by washing it from the substrate.

Further embodiments of the invention provide kits for carrying out the assay of the invention. The kits of the invention can contain specific endonucleases necessary to carry out the assay of the invention, specific guide molecules designed to target one or more target genomic regions, a computer software program designed to process the sequencing data obtained from the assay and optionally, materials that provide instructions to perform the assay. In one embodiment, the kit of the invention comprises:
a) one or more guide molecules as defined in the claims,
b) one or more bridge oligos designed to capture one or more target genomic regions as defined in the claims.

The kit can further comprise one or more programmable endonucleases.

In addition, the kits can comprise primers for PCR or RCA reaction of circularized target genomic regions, DNA ligase, polymerase and other reagents for PCR or RCA, restriction endonucleases, for example, rare-cutters to cleave concatenated copies of the target genomic regions, and sequencing reagents.

In certain embodiments, the kit of the invention can be customized for one or more specific target genomic regions. For example, a user may provide the sequences of one or more target genomic regions and a kit can be produced to carry out the assay of the invention for the one or more target sequences.

Following are examples which illustrate procedures for practicing the invention. These examples should not be construed as limiting. All percentages are by weight and all solvent mixture proportions are by volume unless otherwise noted.

### EXAMPLE 1 - DESIGNING RECOGNITION SITES AND CAPTURING A TARGET GENOMIC REGION

This example describes a typical procedure for capturing and analyzing a target genomic region according to the materials and methods disclosed herein. For example, the following sequence is identified for analysis in this example.

5’ACCCACTGTTGAGAGTCAGTGGCAAGAGAAGTCTCGTCTTTTACGTCCCGTATC
AAAATGAGTGTACAATACAT[A/G]TCTATGTGCGAGTGAGA|GCACGGGTCTGGGC
CTGGAGAGTGGACCACCTACAATTGGCCATTTCGGTTTGCGGAAGCTGTCAAGTC
AACGCGAGTCCTAG[G/A]ATCTCATAGTCTTCGCATTAACCCGTATTAAGTGGACT
CGCCTACAGTTTGTCTTATGCTAGCAACCCAGGCA[T/C]AGTCTGTACGCGCGATC
GTCCACTGG|TAGTGGGGTCCTTCCTGGAGTGT[C/G]GTATGGGCCATCCGGTCTAC
CTTACTAACTTAGGCTTTAAGCGCATTTCTATGTGCGTGAGGTGTCGCATTCATAC
TTAGTCTGGTCCTAAGTCTGTCACC 3’ (SEQ ID NO: 1).

The sequence of SEQ ID NO: 1 provides the sequence of the non-complementary strand, i.e., the gRNA binds to the opposite strand. Hence, the recognition sites are present on the opposite strand and the regions corresponding to the recognition sites are bolded and italicized, NGG PAM motif is underlined and the sites for cleavage by Cas9 endonuclease are indicated by vertical bars. A first gRNA is designed to bind to the first recognition site having the sequence that is reverse complementary to the sequence of TCTATGTGCGAGTGAGAGCA (SEQ ID NO: 2) and the second gRNA is designed to bind to the second recognition site having the sequence that is reverse complementary to the sequence of GCGCGATCGTCCACTGGTAG (SEQ ID NO: 3). The two recognition sites flank a genomic sequence having certain single nucleotide polymorphisms (SNPs) indicated by the nucleotides in the solid brackets. Cas9 mediated cleavage of this sequence will produce a sequence of 184 bp. Sequencing this fragment will provide information about the SNPs present within this region.

Cas9 mediated cleavage of a genetic material containing the sequence of SEQ ID NO: 1 will produce the following fragment, which is the target genomic region.

5'GCACGGGTCTGGGCCTGGAGAGTGGACCACCTACAATTGGCCATTTCGG
TTTGCGGAAGCTGTCAAGTCAACGCGAGTCCTAG[G/A]ATCTCATAGTCTTCGCAT
TAACCCGTATTAAGTGGACTCGCCTACAGTTTGTCTTATGCTAGCAACCCAGGCA[
T/C]AGTCTGTACGCGCGATCGTCCACTGG 3' (SEQ ID NO: 4).

To create a bridge oligo to capture the target genomic region of SEQ ID NO: 4, the following sequences from the ends of the target genomic region are selected, as underlined in the sequence reproduced above:
5' GCACGGGTCTGGGCCTGGAGAGTGGACCAC (SEQ ID NO: 5)
5' GCATAGTCTGTACGCGCGATCGTCCACTGG (SEQ ID NO: 6).

Sequences complementary to these sequences are produced for the ends of the bridge oligo to capture the target genomic region of SEQ ID NO: 4.
5' GTGGTCCACTCTCCAGGCCCAGACCCGTGC 3' (SEQ ID NO: 7)
5' CCAGTGGACGATCGCGCGTACAGACTATGC 3' (SEQ ID NO: 8)

SEQ ID NOs: 7 and 8 are connected to create a bridge oligo of the following sequence:

5’GTGGTCCACTCTCCAGGCCCAGACCCGTGCCCAGTGGACGATCGCGCGT
ACAGACTATGC 3’ (SEQ ID NO: 9).

It should be understood that the examples and embodiments described herein are for illustrative purposes only.

### REFERENCES

Dahl, F., Stenberg, J., Fredriksson, S., Welch, K., Zhang, M., Nilsson, M., et al. (2007). Multigene amplification and massively parallel sequencing for cancer mutation discovery. Proc. Natl. Acad. Sci. U. S. A. 104, 9387-92. doi:10.1073/pnas.0702165104.

Fredriksson, S., Banér, J., Dahl, F., Chu, A., Ji, H., Welch, K., et al. (2007). Multiplex amplification of all coding sequences within 10 cancer genes by Gene-Collector. Nucleic Acids Res. 35, e47. doi:10.1093/nar/gkm078.

Hegge, J. W., Swarts, D. C., and van der Oost, J. (2017). Prokaryotic Argonaute proteins: novel genome-editing tools? Nat. Rev. Microbiol. 16, 5-11. doi:10.1038/nrmicro.2017.73.

Hu, J. H., Miller, S. M., Geurts, M. H., Tang, W., Chen, L., Sun, N., et al. (2018). Evolved Cas9 variants with broad PAM compatibility and high DNA specificity. Nature 556, 57-63. doi:10.1038/nature26155.

Jinek, M., Chylinski, K., Fonfara, I., Hauer, M., Doudna, J. A., and Charpentier, E. (2012). A programmable dual-RNA-guided DNA endonuclease in adaptive bacterial immunity. Science 337, 816-21. doi:10.1126/science.1225829.

Nilsson, M., Malmgren, H., Samiotaki, M., Kwiatkowski, M., Chowdhary, B. P., and Landegren, U. (1994). Padlock probes: circularizing oligonucleotides for localized DNA detection. Science 265, 2085-8. Available at: http://www.ncbi.nlm.nih.gov/pubmed/7522346 [Accessed May 1, 2018].

Swarts, D. C., Hegge, J. W., Hinojo, I., Shiimori, M., Ellis, M. A., Dumrongkulraksa, J., et al. (2015). Argonaute of the archaeon Pyrococcus furiosus is a DNA-guided nuclease that targets cognate DNA. Nucleic Acids Res. 43, 5120-5129. doi:10.1093/nar/gkv415.

Varshney, G. K., and Burgess, S. M. (2016). DNA-guided genome editing using structure-guided endonucleases. Genome Biol. 17, 187. doi:10.1186/s13059-016-1055-4.

Xu, S., Cao, S., Zou, B., Yue, Y., Gu, C., Chen, X., et al. (2016). An alternative novel tool for DNA editing without target sequence limitation: the structure-guided nuclease. *Genome Biol.* 17, 186. doi:10.1186/s13059-016-1038-5.

## Claims

1. A method for capturing a target genomic region from a genetic material, the method comprising the steps of:
a) cleaving the target genomic region from the genetic material using one or more endonucleases having a first recognition site and a second recognition site, each recognition site comprising a sequence of between 10 and 80 nucleotides, wherein the recognition sites flank the target genomic region,
b) denaturing the cleaved genetic material into single stranded form, and
c) capturing the target genomic region in the single stranded form by hybridizing the target genomic region to a bridge oligo, the bridge oligo comprising sequences at the 3' and 5' ends that hybridize to the 3' and 5' ends, respectively, of the target genomic region in the single stranded form.

2. The method of claim 1, wherein each of the first recognition site and the second recognition site comprises a sequence of between 10 and 30 nucleotides and the first and the second recognition sites flank the target genomic region.

3. The method of claim 1 or 2, wherein the one or more endonucleases that cleave the genetic material at the specific recognition sites are restriction endonucleases or programmable endonucleases, preferably with restriction endonucleases being meganucleases and with programmable endonucleases being selected from Clustered Regularly Interspaced Short Palindromic Repeats (CRISPR) associated protein 9 endonucleases (Cas9 endonucleases), Cpf1, C2c1, C2c2, C2c, RNA- or DNA-guided Argonaute proteins and structure-guided endonucleases, with the programmable endonucleases optionally comprising a first endonuclease that cuts DNA at the first recognition site based on a first guide molecule having a sequence complementary to the first recognition site and a second programmable endonuclease that cuts DNA at the second recognition site based on a second guide molecule having a sequence complementary to the second recognition site.

4. The method of any of the preceding claims, wherein
the bridge oligo is a single stranded oligonucleotide comprising sequences at the 3' and 5' ends that hybridize to the sequences at the 3' and the 5' ends, respectively, of the single stranded target genomic region, or
the bridge oligo is a double stranded oligonucleotide having 3' and 5' end overhangs that hybridize to the sequences at the 3' and the 5' ends, respectively, of the single stranded target genomic region, and
optionally the bridge oligo further comprises restriction sites specific for rare-cutter restriction endonucleases, cleavage sites for programmable endonucleases, and/or primer binding sequences; and/or the bridge oligo is immobilized on a solid substrate or is biotinylated.

5. The method of any of the preceding claims, wherein the first and the second recognition sites are present on the same strand of the genetic material, or the first and the second recognition sites are present on different strands of the genetic material.

6. The method of any of the preceding claims, wherein denaturing the cleaved genetic material into the single stranded form comprises subjecting the cleaved genetic material to a temperature of between: 75°C to 115°C, 80°C to 110°C, 85°C and 105°C, 90°C and 100°C, or about 95°C.

7. The method of any of preceding claims, further comprising analyzing the target genomic region, comprising:
d) ligating the free ends of the single stranded target genomic region hybridized to the bridge oligo to produce a single stranded circular target genomic region that is hybridized to the bridge oligo,
e) optionally, degrading non-circularized genetic material,
f) optionally, amplifying the target genomic region by nucleic acid amplification to produce multiple copies of the target genomic region, and
g) analyzing the amplified target genomic region.

8. The method of claim 7, comprising degrading non-circularized genetic material by a combination of exonucleases that degrade the nucleic acids from their exposed 3' or 5' terminus, thereby leaving only the circularized target genomic regions hybridized to the bridge oligo.

9. A method for capturing a plurality of target genomic regions from a genetic material, comprising:
a) cleaving a plurality of target genomic regions from the genetic material using a plurality of pairs of endonucleases, each pair of endonucleases having a first recognition site and a second recognition site, each recognition site comprising a sequence of a minimum of between 17 and 24 nucleotides, wherein each pair of recognition sites flanks a target genomic region from the plurality of target genomic regions,
b) denaturing the cleaved genetic material to single stranded form, and
c) capturing the plurality of target genomic regions in the single stranded form by hybridizing the plurality of target genomic region to a plurality of bridge oligos, wherein each bridge oligo comprises sequences at the 3' and 5' ends that hybridize to the 3' and 5' ends, respectively, of a target genomic region from the plurality of target genomic regions in single stranded form.

10. The method of claim 9, further comprising analyzing the plurality of target genomic regions, comprising:
d) ligating the free ends of the single stranded target genomic regions hybridized to the plurality of bridge oligos to produce a plurality of single stranded circular target genomic regions, each single stranded circular target genomic region containing a target genomic region from the plurality of target genomic regions hybridized to the corresponding bridge oligo,
e) optionally, removing non-circularized genetic material,
f) optionally, amplifying the plurality of target genomic regions by nucleic acid amplification to produce multiple copies of each target genomic region, and
g) analyzing the amplified target genomic regions.

11. The method of claim 9 or 10, wherein the plurality of bridge oligos is immobilized onto a solid substrate and the method optionally comprising amplifying the plurality of target genomic regions by nucleic acid amplification to produce multiple copies of each target genomic region.

12. The method of any one of claims 7-11, comprising
amplifying the target genomic region by polymerase chain reaction (PCR) to produce multiple copies of the target genomic region, the PCR primers being optionally specific to the target regions or, preferably, being universal to all targets by designing them to bind to a common region introduced by the bridge oligo, or
amplifying target genomic regions by a rolling circle amplification (RCA) reaction to produce multiple concatenated copies of target genomic regions, with optionally the bridge oligos being used as a primer for the RCA reaction to produce multiple concatenated copies of each of the target genomic regions in single stranded form, or the bridge oligos and/or one or more additional primers are used for the RCA reaction to produce multiple concatenated copies of target genomic regions in double stranded form.

13. The method of any of claims 7-12, wherein the analyzing comprises detecting the target genomic regions, preferably by contacting the target genomic region with a labeled probe that specifically binds to the target genomic region or a substance that binds to the amplified target molecules; or sequencing the target genomic regions, preferably by nanopore sequencing, reversible dye-terminator sequencing or Single Molecule Real-Time (SMRT) sequencing.

14. A kit comprising:
a) one or more guide molecule(s) to direct one or more endonuclease(s) to one or more target genomic region(s), said one or more endonuclease(s) having a first recognition site and a second recognition site, each recognition site comprising a sequence of between 10 and 80 nucleotides, and
b) one or more bridge oligo(s) designed to capture one or more cleaved target genomic region(s) produced by said one or more endonuclease(s), the one or more bridge oligo(s) having sequences at the 3' and the 5' ends that hybridize to the sequences at the 3' and 5' ends, respectively, of the one or more cleaved target genomic region(s) produced by said one or more endonuclease(s).

15. The kit according to claim 14, wherein
the one or more endonuclease(s) is one or more programmable endonuclease(s) and/or
the one or more bridge oligo(s) are immobilized on a solid substrate; and/or
the kit further comprising one or more of:
primers designed to amplify circularized target genomic regions in a PCR or RCA reaction, ligase polymerase, and one or more rare-cutter restriction endonucleases.

## Patentansprüche

1. Ein Verfahren zur Gewinnung einer genomischen Zielregion aus einem genetischen Material, wobei das Verfahren die folgenden Schritte umfasst:
a) Ausschneiden der genomischen Zielregion aus dem genetischen Material mit Hilfe von einer oder mehreren Endonukleasen mit einer ersten Erkennungsstelle und einer zweiten Erkennungsstelle, wobei jede Erkennungsstelle eine Sequenz von zwischen 10 und 80 Nukleotiden umfasst, wobei die Erkennungsstellen die genomische Zielregion flankieren,
b) Denaturieren des ausgeschnittenen genetischen Materials zur einzelsträngigen Form, und
c) Gewinnen der genomischen Zielregion in der einzelsträngigen Form mittels Hybridisierung der genomischen Zielregion an ein Brückenoligo, wobei das Brückenoligo am 3'- und 5'-Ende Sequenzen umfasst, die mit dem 3'- bzw. 5'-Ende der genomischen Zielregion in der einzelsträngigen Form hybridisieren.

2. Das Verfahren nach Anspruch 1, wobei jede der ersten Erkennungsstelle und der zweiten Erkennungsstelle eine Sequenz von zwischen 10 und 30 Nukleotiden umfasst und die erste und die zweite Erkennungsstellen die genomische Zielregion flankieren.

3. Das Verfahren nach Anspruch 1 oder 2, wobei die eine oder mehreren Endonukleasen, die das genetische Material an den spezifischen Erkennungsstellen schneiden, Restriktionsendonukleasen oder programmierbare Endonuklease sind, wobei vorzugsweise die Restriktionsendonukleasen Meganukleasen sind, und die programmierbaren Endonukleasen aus Clustered Regularly Interspaced Short Palindromic Repeats (CRISPR) associated protein 9-Endonukleasen (Cas 9-Endonukleasen), Cpf1, C2c1, C2c2, C2c, RNA- oder DNA-gelenkten Argonautenproteinen und strukturgelenkten Endonukleasen ausgewählt sind, wobei die programmierbaren Endonukleasen optional eine erste Endonuklease umfassen, die die DNA an der ersten Erkennungsstelle auf Grundlage eines ersten Lenkmoleküls schneidet, das eine zu der ersten Erkennungsstelle komplementäre Sequenz hat, und eine zweite programmierbare Endonuklease, die die DNA an der zweiten Erkennungsstelle auf Grundlage eines zweiten Lenkmoleküls schneidet, das eine Sequenz hat, die komplementär zu der zweiten Erkennungsstelle ist.

4. Das Verfahren nach einem der vorhergehenden Ansprüche, wobei
das Brückenoligo ein einzelsträngiges Oligonukleotid ist, das an den 3'- und 5'-Enden Sequenzen umfasst, die mit den 3'- bzw. 5'-Enden der einzelsträngigen genomischen Zielregion hybridisieren, oder
das Brückenoligo ein doppelsträngiges Oligonukleotid mit Überhängen an den 3'- und 5'-Enden ist, die mit den 3'- bzw. 5'-Enden der einzelsträngigen genomischen Zielregion hybridisieren, und
optional das Brückenoligo ferner Restriktionsschnittstellen, die spezifisch für selten schneidende Restriktionsendonukleasen sind, Schnittstellen für programmierbare Endonukleasen, und/oder Primerbindungssequenzen umfasst; und/oder das Brückenoligo auf einem festen Substrat immobilisiert ist oder biotinyliert ist.

5. Das Verfahren nach einem der vorhergehenden Ansprüche, wobei sich die ersten und zweiten Erkennungsstellen auf dem gleichen Strang des genetischen Materials befinden, oder sich die ersten und zweiten Erkennungsstellen auf verschiedenen Strängen des genetischen Materials befinden.

6. Das Verfahren nach einem der vorhergehenden Ansprüche, wobei das Denaturieren des geschnittenen genetischen Materials in die einzelsträngige Form umfasst, dass das geschnittene genetische Material einer Temperatur von zwischen 75°C bis 115°C, 80°C bis 110°C, 85°C and 105°C, 90°C and 100°C, oder etwa 95°C ausgesetzt wird.

7. Das Verfahren nach einem der vorhergehenden Ansprüche, ferner umfassend das Analysieren der genomischen Zielregion, umfassend:
d) Ligieren der freien Enden der einzelsträngigen genomischen Zielregion, die an das Brückenoligo hybridisiert ist, um eine einzelsträngige zirkuläre genomische Zielregion herzustellen, die an das Brückenoligo hybridisiert ist,
e) optional, Abbauen nicht-zirkulären genetischen Materials,
f) optional, Amplifizieren der genomischen Zielregion mit Hilfe von Nukleinsäureamplifikation, um multiple Kopien der genomischen Zielregion herzustellen, und
g) Analysieren der amplifizierten genomischen Zielregion.

8. Das Verfahren nach Anspruch 7, umfassend das Abbauen nicht-zirkulären genetischen Materials mit Hilfe einer Kombination an Exonukleasen, die Nukleinsäuren von ihrem exponierten 3'- oder 5'-Terminus her abbauen, wodurch nur die zirkularisierte genomische Zielregionen, die an das Brückenoligo hybridisiert sind, übrig bleiben.

9. Ein Verfahren zur Gewinnung einer Vielzahl genomischer Zielregionen aus einem genetischen Material, umfassend:
a) Ausschneiden einer Vielzahl an genomischen Zielregionen aus dem genetischen Material mit Hilfe einer Vielzahl an Endonukleasepaaren, wobei jedes Endonukleasepaar eine erste Erkennungsstelle und eine zweite Erkennungsstelle hat, jede Erkennungsstelle eine Sequenz mit mindestens von zwischen 17 und 24 Nukleotiden umfasst, wobei jedes Erkennungsstellenpaar eine genomische Zielregion der Vielzahl an genomischen Zielregionen flankiert,
b) Denaturieren des genetischen Materials in die einzelsträngige Form, und
c) Gewinnen der Vielzahl an genomischen Zielregionen in der einzelsträngigen Form durch Hybridisieren der Vielzahl an genomischen Zielregionen mit einer Vielzahl an Brückenoligos, wobei jedes Brückenoligo an den 3'- und 5'-Enden Sequenzen umfasst, die mit den 3'- bzw. 5'-Enden einer genomischen Zielregion aus der Vielzahl an genomischen Zielregionen in der einzelsträngigen Form hybridisieren.

10. Das Verfahren nach Anspruch 9, ferner umfassend das Analysieren der Vielzahl an genomischen Zielregionen, umfassend:
d) Ligieren der freien Enden der einzelsträngigen genomischen Zielregionen, die an die Vielzahl an Brückenoligos hybridisiert sind, um eine Vielzahl an einzelsträngigen zirkulären genomischen Zielregionen herzustellen, wobei jede einzelsträngige zirkuläre genomische Zielregion eine genomische Zielregion der Vielzahl der an das entsprechende Brückenoligo hybridisierten genomischen Zielregionen enthält,
e) optional, Entfernen nicht-zirkulären genetischen Materials,
f) optional, Amplifizieren der Vielzahl an genomischen Zielregionen mit Hilfe von Nukleinsäureamplifikation, um multiple Kopien einer jeden genomischen Zielregion herzustellen, und
g) Analysieren der amplifizierten genomischen Zielregionen.

11. Das Verfahren nach Anspruch 9 oder 10, wobei die Vielzahl an Brückenoligos auf einem festen Substrat immobilisiert ist und das Verfahren optional das Amplifizieren der Vielzahl an genomischen Zielregionen mit Hilfe von Nukleinsäureamplifikation umfasst, um multiple Kopien einer jeden genomischen Zielregion herzustellen.

12. Das Verfahren nach einem der Ansprüche 7-11, umfassend
Amplifizieren der genomischen Zielregion mittels Polymerasekettenreaktion (PCR) um multiple Kopien der genomischen Zielregion herzustellen, wobei die PCR-Primer optional spezifisch für die Zielregionen sind oder, vorzugsweise, universell für alle Ziele sind, in dem man sie so entwirft, dass sie an eine gemeinsame Region binden, die durch das Brückenoligo eingeführt wurde, oder
Amplifizieren der genomischen Zielregionen mit Hilfe einer Rolling-Circle-Amplifikation (RCA)-Reaktion um multiple konkatenierte Kopien der genomischen Zielregionen herzustellen, wobei die Brückenoligos optional als Primer für die RCA-Reaktion verwendet werden um multiple konkatenierte Kopien einer jeden der genomischen Zielregionen in einzelsträngiger Form herzustellen, oder die Brückenoligos und/oder einer oder mehrere zusätzliche Primer für die RCA-Reaktion verwendet werden, um multiple konkatenierte Kopien der genomischen Zielregionen in doppelsträngiger Form herzustellen.

13. Das Verfahren nach einem der Ansprüche 7-12, wobei das Analysieren das Nachweisen der genomischen Zielregionen umfasst, vorzugsweise in dem man die genomische Zielregion mit einer markierten Sonde in Kontakt bringt, die spezifisch an die genomische Zielregion bindet, oder mit einer Substanz, die an die vervielfältigten Zielmoleküle bindet; oder Sequenzieren der genomischen Zielregionen, vorzugsweise mittels Nanopore-Sequenzierung, Reversible Dye-Terminator-Sequenzierung, oder Single Molecule Real-time (SMRT)-Sequenzierung.

14. Ein Kit umfassend:
a) ein oder mehrere Lenkmolekül(e) um eine oder mehrere Endonuklease(n) zu einer oder mehreren genomischen Zielregion(en) zu führen, wobei die eine oder mehrere Endonuklease(n) eine erste Erkennungsstelle und eine zweite Erkennungsstelle haben, jede Erkennungsstelle eine Sequenz von zwischen 10 und 80 Nukleotiden umfasst, und
b) ein oder mehrere Brückenoligo(s), das/die so entworfen ist/sind, dass es/sie ein oder mehrere ausgeschnittene genomische Zielregion(en), hergestellt von der einen oder den mehreren Endonuklease(n), zu fangen vermag, wobei das eine oder die mehreren Brückenoligo(s) Sequenzen an den 3'- und 5'-Enden haben, die mit den Sequenzen an den 3'- bzw. 5'-Enden der einen oder den mehreren ausgeschnittenen genomischen Zielregion(en) hybridisieren, die mittels der einen oder den mehreren Endonuklease(b) hergestellt wurden.

15. Der Kit nach Anspruch 14, wobei
die eine oder die mehreren Endonuklease(n) eine oder mehrere programmierbare Endonuklease(n) ist/sind und/oder
das eine oder die mehreren Brückenoligo(s) auf einem festen Substrat immobilisiert sind; und/oder
der Kit ferner eines oder mehrere des Folgenden umfasst:
Primer, die so entworfen sind, dass sie zirkuläre genomische Regionen in einer PCR- oder RCA-Reaktion amplifizieren, Ligasepolymerase, und eine oder mehrere selten schneidende Restriktionsendonukleasen.

## Revendications

1. Méthode pour capturer une région génomique cible à partir d'un matériau génétique, la méthode comprenant les étapes de :
a) coupure de la région génomique cible à partir du matériau génétique par utilisation d'une ou plusieurs endonucléases ayant un premier site de reconnaissance et un deuxième site de reconnaissance, chaque site de reconnaissance comprenant une séquence d'entre 10 et 80 nucléotides, dans laquelle les sites de reconnaissance flanquent la région génomique cible,
b) dénaturation le matériau génétique coupé en une forme simple brin, et
c) capture de la région génomique cible sous la forme simple brin par hybridation de la région génomique cible à un oligo pont, l'oligo pont comprenant des séquences aux extrémités 3' et 5' qui s'hybrident aux extrémités 3' et 5', respectivement, de la région génomique cible sous la forme simple brin.

2. Méthode selon la revendication 1, dans laquelle chacun parmi le premier site de reconnaissance et le deuxième site de reconnaissance comprend une séquence d'entre 10 et 30 nucléotides, et les premier et deuxième sites de reconnaissance flanquent la région génomique cible.

3. Méthode selon la revendication 1 ou 2, dans laquelle la ou les endonucléases qui coupent le matériau génétique au niveau des sites de reconnaissance spécifiques sont des endonucléases de restriction ou des endonucléases programmables, de préférence les endonucléases de restriction sont des méganucléases et les endonucléases programmables sont choisies parmi les endonucléases protéiques 9 associées à des répétitions courtes palindromiques groupées et régulièrement espacées (CRISPR) (endonucléases Cas9), Cfp1, C2c1, C2c2, C2c, les protéines argonautes guidées par l'ARN ou l'ADN et les endonucléases guidées par la structure, les endonucléases programmables comprenant éventuellement une première endonucléase qui coupe l'ADN au niveau du premier site de reconnaissance sur la base d'une première molécule de guidage ayant une séquence complémentaire du premier site de reconnaissance et une deuxième endonucléase programmable qui coupe l'ADN au niveau du deuxième site de reconnaissance sur la base d'une deuxième molécule de guidage ayant une séquence complémentaire du deuxième site de reconnaissance.

4. Méthode selon l'une quelconque des revendications précédentes, dans laquelle
l'oligo pont est un oligonucléotide simple brin comprenant des séquences aux extrémités 3' et 5' qui s'hybrident aux séquences aux extrémités 3' et 5', respectivement, de la région génomique cible simple brin, ou
l'oligo pont est un oligonucléotide double brin ayant des surplombs d'extrémités 3' et 5' qui s'hybrident aux séquences aux extrémités 3' et 5', respectivement, de la région génomique cible simple brin, et
éventuellement l'oligo pont comprend en outre des sites de restriction spécifiques d'endonucléases de restriction à site de coupure rare, des sites de coupure pour endonucléases programmables, et/ou des séquences de liaison à une amorce ; et/ou l'oligo pont est immobilisé sur un substrat solide ou est biotinylé.

5. Méthode selon l'une quelconque des revendications précédentes, dans laquelle les premier et deuxième sites de reconnaissance sont présents sur le même brin du matériau génétique, ou bien les premier et deuxième sites de reconnaissance sont présents sur des brins différents du matériau génétique.

6. Méthode selon l'une quelconque des revendications précédentes, dans laquelle la dénaturation en la forme simple brin du matériau génétique coupé comprend la soumission du matériau génétique coupé à une température comprise : entre 75 °C et 115 °C, entre 80 °C et 110 °C, entre 85 °C et 105 °C, entre 90 °C et 100 °C, ou d'environ 95 °C.

7. Méthode selon l'une quelconque des revendications précédentes, comprenant en outre l'analyse de la région génomique cible, comprenant :
d) la ligature des extrémités libres de la région génomique cible simple brin hybridée à l'oligo pont pour produire une région génomique cible circulaire simple brin qui est hybridée à l'oligo pont,
e) éventuellement la dégradation de matériau génétique non circularisé,
f) éventuellement l'amplification de la région génomique cible par amplification d'acide nucléique pour produire de multiples copies de la région génomique cible, et
g) l'analyse de la région génomique cible amplifiée.

8. Méthode selon la revendication 7, comprenant la dégradation de matériau génétique non circularisé par une combinaison d'exonucléases qui dégradent les acides nucléiques à partir de leur extrémité 3' ou 5' exposée, en laissant ainsi uniquement les régions génomiques cibles circularisées hybridées à l'oligo pont.

9. Méthode pour capturer une pluralité de régions génomiques cibles à partir d'un matériau génétique, comprenant :
a) la coupure d'une pluralité de régions génomiques cibles à partir du matériau génétique par utilisation d'une pluralité de paires d'endonucléases, chaque paire d'endonucléases ayant un premier site de reconnaissance et un deuxième site de reconnaissance, chaque site de reconnaissance comprenant une séquence faisant au minimum entre 17 et 24 nucléotides, dans laquelle chaque paire de sites de reconnaissance flanque une région génomique cible parmi la pluralité de régions génomiques cibles,
b) la dénaturation en une forme simple brin du matériau génétique coupé, et
c) la capture de la pluralité de régions génomiques cibles sous la forme simple brin par hybridation de la pluralité de régions génomiques cibles à une pluralité d'oligos ponts, dans laquelle chaque oligo pont comprend des séquences aux extrémités 3' et 5' qui s'hybrident aux extrémités 3' et 5', respectivement, d'une région génomique cible parmi la pluralité de régions génomiques cibles sous la forme simple brin.

10. Méthode selon la revendication 9, comprenant en outre l'analyse de la pluralité de régions génomiques cibles, comprenant :
d) la ligature des extrémités libres des régions génomiques cibles simple brin hybridées à la pluralité d'oligos ponts pour produire une pluralité de régions génomiques cibles circulaires simple brin, chaque région génomique cible circulaire simple brin contenant une région génomique cible parmi la pluralité de régions génomiques cibles qui est hybridée à l'oligo pont correspondant,
e) éventuellement l'élimination de matériau génétique non circularisé,
f) éventuellement l'amplification de la pluralité de régions génomiques cibles par amplification d'acide nucléique pour produire de multiples copies de chaque région génomique cible, et
g) l'analyse des régions génomiques cibles amplifiées.

11. Méthode selon la revendication 9 ou 10, dans laquelle la pluralité d'oligos ponts est immobilisée sur un substrat solide, la méthode comprenant éventuellement l'amplification de la pluralité de régions génomiques cibles par amplification d'acide nucléique pour produire de multiples copies de chaque région génomique cible.

12. Méthode selon l'une quelconque des revendications 7 à 11, comprenant
l'amplification de la région génomique cible par amplification en chaîne par polymérase (PCR) pour produire de multiples copies de la région génomique cible, les amorces de PCR étant éventuellement spécifiques des régions cibles ou, de préférence, étant universelles pour toutes les cibles en étant conçues pour se lier à une région commune introduite par l'oligo pont, ou
l'amplification des régions génomiques cibles par amplification en cercle roulant (RCA) pour produire de multiples copies concaténées de régions génomiques cibles, les oligos ponts étant éventuellement utilisés en tant qu'amorces pour la réaction RCA pour produire de multiples copies concaténées de chacune des régions génomiques cibles sous la forme simple brin, ou bien les oligos ponts et/ou une ou plusieurs amorces additionnelles étant utilisés pour la réaction RCA pour produire de multiples copies concaténées de régions génomiques cibles sous la forme double brin.

13. Méthode selon l'une quelconque des revendications 7 à 12, dans laquelle l'analyse comprend la détection des régions génomiques cibles, de préférence par mise en contact de la région génomique cible avec une sonde marquée qui se lie spécifiquement à la région génomique cible ou une substance qui se lie aux molécules cibles amplifiées ; ou le séquençage des régions génomiques cibles, de préférence par séquençage par nanopores, séquençage par terminateurs fluorescents réversibles ou séquençage en temps réel de molécule unique (SMRT).

14. Trousse comprenant :
a) une ou plusieurs molécules de guidage pour diriger une ou plusieurs endonucléases vers une ou plusieurs régions génomiques cibles, lesdites une ou plusieurs endonucléases ayant un premier site de reconnaissance et un deuxième site de reconnaissance, chaque site de reconnaissance comprenant une séquence d'entre 10 et 80 nucléotides, et
b) un ou plusieurs oligos ponts conçus pour capturer une ou plusieurs régions génomiques cibles coupées produites par lesdites une ou plusieurs endonucléases, le ou les oligos ponts ayant des séquences aux extrémités 3' et 5' qui s'hybrident aux séquences aux extrémités 3' et 5', respectivement, de la ou des régions génomiques cibles coupées produites par lesdites une ou plusieurs endonucléases.

15. Trousse selon la revendication 14, dans laquelle
la ou les endonucléases sont une ou plusieurs endonucléases programmables et/ou le ou les oligos ponts sont immobilisés sur un substrat solide ; et/ou
la trousse comprenant en outre une ou plusieurs parmi :
des amorces conçues pour amplifier des régions génomiques cibles circularisées dans une réaction PCR ou RCA, une ligase polymérase, et une ou plusieurs endonucléases de restriction à site de coupure rare.
